# EUROPEAN PATENT SPECIFICATION

(11) **EP 3 711 669 B1**
(45) Date of publication and mention of the grant of the patent: **18.08.2021**
(21) Application number: 20163204.9
(22) Date of filing: 13.03.2020
(51) Int. Cl.: A61B 5/15, A61B 5/151, A61B 5/157

(54) **DEVICE FOR BLOOD COLLECTION AND ANALYSIS**
GERÄT FÜR BLUTPROBEENTNAHME UND ANALYSE
DISPOSITIF DE COLLECTE DE SANG ET ANALYSE

(30) Priority: 15.03.2019 PL 42929019
(43) Date of publication of application: 23.09.2020
(73) Proprietor: Uniwersytet Medyczny w Lodzi, 90-419 Lodz (PL)
(72) Inventor: GAWRON-SKARBEK, Anna, 98-290 Warta (PL); GRABSKA-KOBYLECKA, Izabela, LODZ (PL)
(74) Representative: Godlewski, Piotr

(56) References cited:
- EP-A1- 2 253 272
- EP-A1- 2 757 952
- EP-A2- 1 903 942
- WO-A2-2005/018709
- WO-A2-2006/004859
- US-A1- 2010 234 869

## Description

The subject of the invention is a device for analysis of blood parameters, which is used especially during automatic blood collection and analysis of its parameters in non-hospital conditions and without medical personnel. In particular, the subject of the invention can be used in the so-called telemedicine, i.e. to control and monitor a patient staying at home.

Health monitoring devices for measuring blood indicators are known in the art. For example, PL / EP 1903942 T3 discloses a device provided with a lancet for puncturing skin to collect blood, and also having a plurality of indicator strips used for analyzing the blood sample. Individual device elements are placed in the body, which forms the outer device housing. There are two slots in the body. The first slot cooperates with puncturing means attached to the body. They are stretched and then released to puncture the skin, with part of the skin puncturing head passing through the first slot into the body. In addition, an additional housing is connected to the body in which a number of indicator strips for blood analysis are located. From the additional housing, the indicator strips can be led out of the device one by one through a second slot in the body. A variable diaphragm is also connected to the device body. The diaphragm in the first position tightly covers the first and second slots on the body, and in its second position the first and second body slots are exposed, which allows for a blood test to be performed. In addition, the movement of the diaphragm can cause the indicator strips to be pushed from the additional housing to the exit in the second body slot, as well as, if necessary, can cause the indicator strips located on the common support base to be cut off.

Also description of PL / EP 2757952 T3 discloses a device for measuring blood parameters, e.g. glucose level, which can be used without medical personnel. The device has a housing, which forms the base of the device and the device outer walls, the top of which is closed by a cover, part of which is removable. On the front of the device there are inputs for device control, a display and a gap for a part of the user's body to collect a blood sample for analysis. The gap has a moveable diaphragm that can cover all or part of the elongated gap when the device is not in use. The device housing can be distinguished by a cylindrical part in which the shaft for test disks for skin puncturing and analyzing blood indicators is coaxially located. The cylindrical part of the housing is positioned so that in the working position the test discs can pass through the gap in the housing. A driving wheel is also placed in the housing, which wheel is coupled to the shaft so that the shaft can be rotated by the movement of the driving wheel. There are many identical test discs on the shaft, each of which in the cut out part has a lancet for skin puncturing. The cut out part of the disk contains protrusions, on tops of which there are capillary slots that connect the area in the disk in which the blood is collected and the blood indicators are measured - blood glucose level, which is then connected to the contact plates and leading paths. The result of blood indicators is visualized on the device's display via contact plates and leading paths. In using this device, the user puts a body part to the measuring gap, and the test disk rotational movements puncture the user's skin and allow the performance of blood parameters analysis, the results of which are displayed on the device's display. Further rotational movement of the shaft provided with test discs causes replacing the used test disc with a new test disc, which will be used for the next blood parameters analysis. The device for measuring blood indicators is equipped with electronic components, are not shown in detail, which are responsible for its operation.

The object of the invention is to solve the problem that occurs when performing medical treatment and monitoring its effects, wherein the user performs blood parameters analysis at home, without medical personnel and without constant supervision over treatment effects. So far, analysis of blood parameters at home could cause reduced effectiveness of planned therapy due to the lack of sufficient supervision and control over the results. Another feature negatively affecting the results of blood parameters analysis was also the lack of repeatability of such tests at specified and appropriately selected time intervals.

The invention relates to a device for analyzing blood parameters, comprising a housing with an entrance opening, inside of which there is a drive connected to a replaceable module, equipped with puncturing means and means for analyzing blood parameters, and inside of which housing there is provided an electronic control module.

The essence of the invention lies in the fact that the replaceable module is mounted in the housing moveably, while the drive located in the housing is in motional connection with the replaceable module. At the same time, in the replaceable module, one can distinguish at least two segments, each of which is equipped with a moveable diaphragm and thereto assigned puncturing means and blood parameters analyzing means, as well as a terminal connecting the blood parameters analyzing means with an electronic control module. The electronic control module itself is equipped with a communication module.

Preferably, an alarm module is provided in the housing.

It is beneficial for the replaceable module to have the form of a rotary ring in which there are four segments, the output of each of these segments being secured by a moveable diaphragm and oriented towards the inside of the ring.

It is particularly desirable for the ring to be provided with four sockets which form the segments, wherein at the bottom of each socket there is provided a springy element terminated with a puncturing means, while above the springy element a flexible membrane is installed forming a reservoir for blood.

It is also appropriate for the entrance opening of the housing to be provided with a moveable closing.

It is also beneficial for the electronic control module to be equipped with a wireless communication module, preferably in the Bluetooth standard.

It is also advantageous that a pneumatic clamping band is mounted in the housing directly in the entrance opening area.

It is to be known, that the term "the replaceable module is mounted in the housing moveably" should be herein understood in such a way, that a working position of the removable module is set in the device, in which position blood sample is collected, and mounting of the replaceable module in moveable manner allows for the module to move to said working position, in particular, individual segments of said removable module.

The main advantage of the invention, achieved in particular thanks to the replaceable module with puncturing means and blood parameters analyzing means, is to provide the possibility of automated sampling of blood for analysis and the analysis of the sample entirely inside the device. The puncturing process is performed inside the device, and immediately after the puncturing process, the blood sample is analyzed. This allows avoiding that the puncturing process by performed by the user of the device. Blood parameters are also measured inside the device, excluding contact of the blood sample with the surroundings and the need for its transport. What is more, equipping the electronic control module with a communication module allows achieving further advantages, allowing in particular transferring the results of analysis of blood parameters to the doctor or the patient's center of the device user. Thus, by using known devices and methods of wireless communication, it is possible to constantly monitor the results of patient tests within the so-called telemedicine, and in critical cases it allows for the possibility of immediate response and modification of treatment parameters. Also, thanks to the exchangeable module being divided into segments and its moveable attachment in the housing, there is no need to replace this module each time after blood collection, because the replaceable module after collecting the blood sample and performing the analysis moves to the position in which the next segment of the replaceable module is used. Consequently, the replaceable module can be adapted, for example, to perform four measurements per day and can be equipped with four uniformly spaced segments with puncturing means and blood parameters analyzing means. The complete replaceable module will have to be replaced with the next one the following day, which significantly reduces the number of device maintenance tasks that the user must perform. In addition, placing the alarm module in the housing allows to get the functionality associated with the reminding of the need to perform blood parameters analysis, depending on the settings of the electronic control module. The use of an individual diaphragm for each segment, as well as a moveable closing of the entrance opening in the housing further allows for limiting the impact of the external environment on the blood parameters analysis. Thus, the device allows continuous care over the patient with the possibility of reminding them of the need to perform the examination. This improves the effectiveness of the conducted treatment monitoring, while maintaining the ability to perform blood parameters analysis at home, without contact of the blood sample with the environment, and without presence of medical personnel during performing the test. Another advantage of the device is the possibility of its multiple use - only a replaceable module, containing aseptic segments with puncturing means and blood parameters analyzing means, requires replacement.

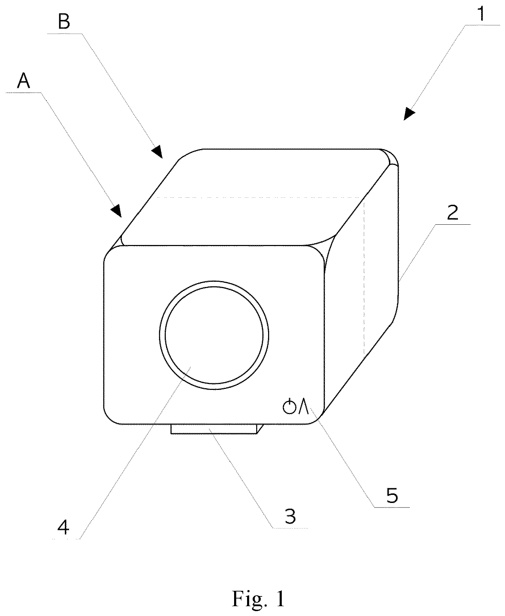

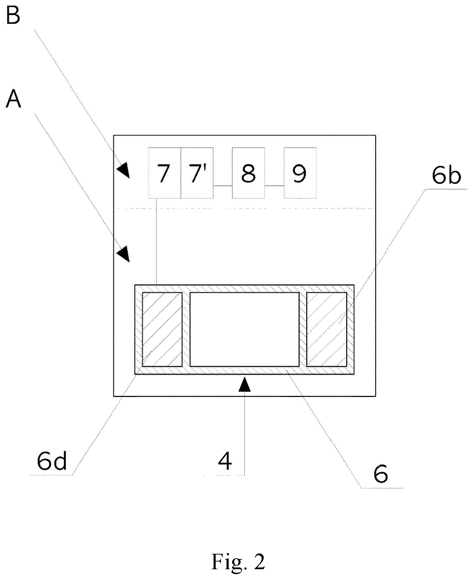

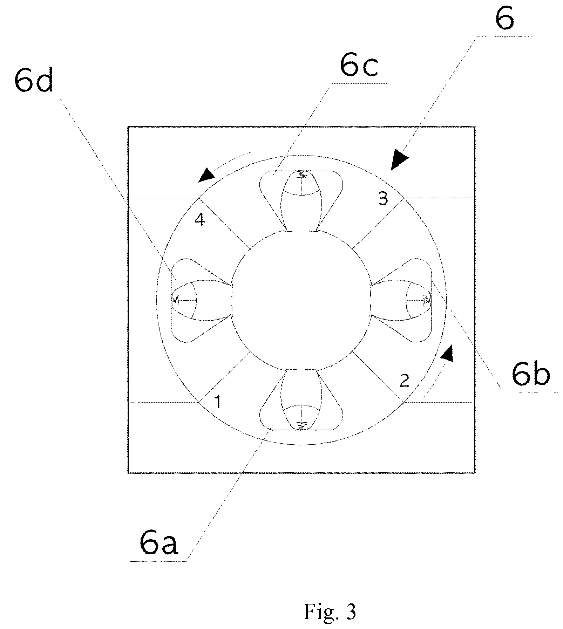

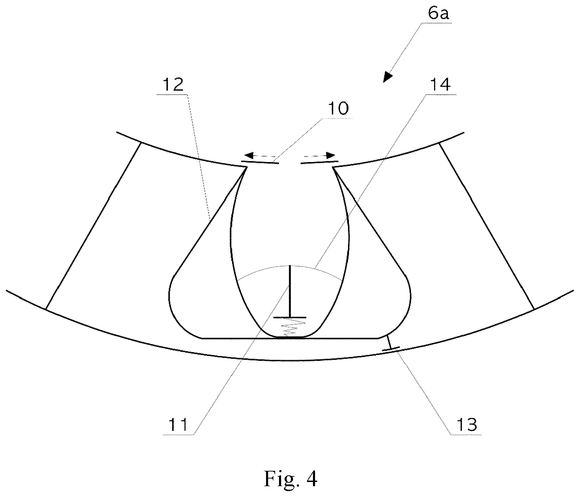

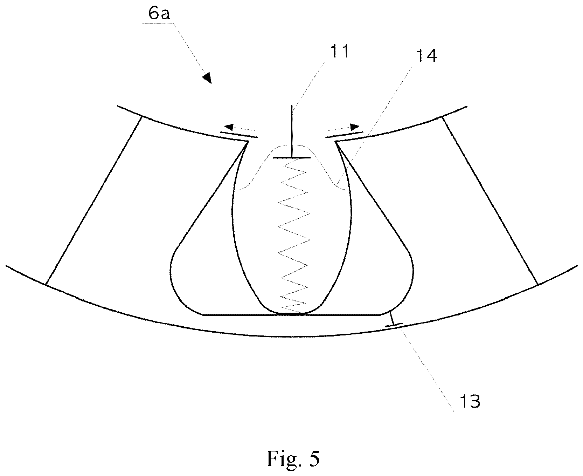

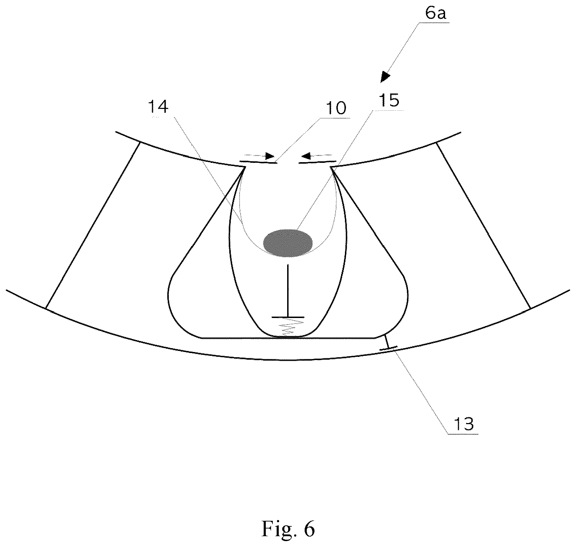

The invention is illustrated in the embodiment on the drawing, in which Fig. 1 shows a perspective view of device for collection and analysis of blood parameters, Fig. 2 is a partial schematic cross-section of device with a block representation of individual components, Fig. 3 is a cross-section of replaceable module, Fig. 4 is a front view of the replaceable module segment with retracted puncturing means, Fig. 5 is the replaceable module segment as in Fig. 4 with the puncturing means in the puncturing position, and Fig. 6 is the replaceable module segment as in Fig. 4 with blood sample taken.

The device 1 for analysis of blood parameters has a housing 2 having an external shape of a cube with rounded tops, in which housing a base 3 is attached in its lower part, for stable positioning of the housing, in the embodiment a mounting suction cup was used to allow for stable fastening of the device 1 on the table. The housing 2 has a round entrance opening 4 having a diameter that allows that a finger can be freely placed therein. The entrance opening 4 defines the frontal part of the device 1 directed towards the user. Optionally, the entrance opening 4 may have a moveable closure, for example in the form of a diaphragm or guillotine, which, however, is not described in detail in the embodiment shown. The moveable closing of the entrance opening 4 limits the contact of the interior of the device 1 with the surroundings. In the front part of the device 1 there are buttons 5 intended for controlling the device. The housing 2 is divided into two parts - the first part A and the second part B of the housing 2. In the first part A of the housing 2 there is a removably mounted replaceable module 6 equipped with puncturing means 11 and blood parameters analyzing means 12. The device 1 users are provided with easy access to the interior of the first part A of the housing 2 to replace the used replaceable module 6. The second part B of the housing 2 contains the components of the device 1 responsible for its correct operation. In this second part B of housing 2, the electronic control module 7 with wireless communication module 7', drive 8 and alarm module 9 are mounted. The housing 2 also has a power module and electrical connections for each module, which are not shown in detail on the drawing. The device 1 can be powered by any method, but the use of rechargeable battery or batteries increases the mobility of the device 1.

The replaceable module 6 is equipped with puncturing means 11 and blood parameters analyzing means 12 and has the form of a ring, which is fixed in the housing 2 interchangeably and movably. The diameter of the inner opening in the ring allows free insertion of the user's finger therein and essentially corresponds to the diameter of the entrance opening 4. The internal opening of the exchangeable ring of the module 6 is placed coaxially with the entrance opening 4. Depending on the method of manufacture, the inner side of the ring may be lined with a material that facilitates stable finger placement or it can also cooperate with a pneumatic clamping band mounted directly in the entrance opening area 4. At the same time, the replaceable module 6 is in motional connection with the drive 8, in such a way that the drive 8 performs the movement of segments 6a, 6b, 6c, 6d of the replaceable module 6, by setting the selected segment 6a, 6b, 6c, 6d to the working position - in which the blood sample is taken. In the embodiment, the segment 6a, i.e. the segment closest to the base 3 of the device 1, is in working position. The segments 6a, 6b, 6c, 6d are evenly spaced on the ring, by 90°. Consequently, the drive 8 performs a rotational movement of the exchangeable module 6, which as a result changes its position by 90°. Each segment 6a, 6b, 6c, 6d has the form of a socket, the outlet of which is equipped with a moveable diaphragm 10 and directed to the inside of the module 6 removable ring. At the bottom of each seating of segment 6a, 6b, 6c, 6d there is a springy element terminated with a lancet for puncturing the patient's skin to obtain a blood sample 15 thus forming puncturing means 11. Above the puncturing means 11 a flexible membrane 14 is mounted in the socket, which membrane after puncturing the patient's skin forms reservoir for blood. In the area of the upper part of the socket, where after the puncture of the skin on the flexible membrane 14 there will be a blood sample 15, as well as on the membrane 14 itself, arranged are surface blood analysis tests thus forming blood parameters analyzing means 12. Each segment 6a, 6b, 6c, 6d also has a terminal 13 connecting the blood parameters analyzing means 12 with the control module 7. The type of blood parameters analyzing means 12 are selected depending on the required type test to be performed at home.

The moveable diaphragm 10 completely separates the interior of each segment 6a, 6b, 6c, 6d from the surroundings, and for the purpose of taking a blood sample, it only opens for the duration of this operation.

The electronic control module 7 is programmable and controls all components of the device 1. In particular, the electronic control module 7 allows for automatic rotation of the replaceable module 6 with the help of the drive 8. At set intervals, e.g. every six hours, the replaceable module 6 and if necessary, the alarm module 9 is activated to remind about the test. This is to ensure regular performance of the blood parameter analysis by the user of the device 1. Alternatively, the rotation of the replaceable module 6 with the participation of the driving 8 may take place after the analysis of the blood sample 15, so that immediately after the end of the test in the device 1 the next segment of the replaceable module 6 is prepared.

When using device 1, the user only has to insert their finger into the entrance opening 4, but it is beneficial for the user to receive appropriate training or instruction on the use of the device 1. In addition, in the convex rim of the entrance opening in the preferred embodiment 4 it is possible to provide the light-emitting element, e.g. in the form of an intermittent red light signal, which could indicate that the device is ready for puncture. It can also be provided that the correct positioning of the finger inside the replaceable module 6 changes the light signal to a continuous green color, which means that the puncturing means 11 will be activated soon. After inserting the finger and contacting the replaceable module 6, the moveable diaphragm 10 of one of the segments 6a, 6b, 6c, 6d which is currently in working position opens. Then, the puncturing means 11 are shot allowing the desired size of the sample of capillary blood to be taken just after the puncture. After the puncture, the blood flows into the reservoir formed by the walls of the upper part of the socket and the flexible membrane 14. The material of the flexible membrane 14 is so chosen that the size of puncture effected the puncturing means 11 is very small and does not cause blood sample 15 to flow through the flexible membrane 14. It is also possible to select the material of the flexible membrane 14 in such a way that, due to its properties, it will close the opening effected by the puncturing means 11. In addition, the use of the flexible membrane 14 allows the pressure difference to be used during a home blood test. With the convex arrangement of the flexible membrane 14 in a position where the puncturing means 11 are at the bottom of the socket, a underpressure can be obtained, and thus the effect of the ejection of the puncturing means 11 can be increased, which further facilitates the return of the puncturing means 11 to the position at the bottom of the socket with, at the same time, the concave arrangement of the flexible membrane 14 as the bottom for the blood sample 15.

Immediately after this step, the user removes their finger from the device 1, the moveable diaphragm 10 closes, and the collected blood sample 15 is subjected to proper analysis using appropriately selected analyzing blood parameters means 12. The result of the blood sample analysis 15 is transferred via terminal 13 to the control module 7, and then using the communication module 7' to the healthcare center or the medical doctor caring for the user. It is particularly advantageous if the communication module 7' is made as a wireless transmission module. In a preferred embodiment, an audible alarm can be provided to signal an incorrect measurement result. In addition, the device may have a memory unit that allows to save the results of the blood sample analysis 15. The results of the analysis itself can be transmitted using the 7' communication module to the patient's phone, where the results can also be presented in a descriptive or graphic form, and then they can be forwarded to the healthcare center or the medical doctor caring for the user. This significantly improves the supervision of the user of the device 1 and the implemented medical treatment, allowing immediate reaction in the event of abnormal results or treatment end points.

## Claims

1. A device for collection and analysis of blood parameters, comprising a housing with an entrance opening, inside of which there is provided a drive connected to a replaceable module equipped with puncturing means and blood parameters analyzing means, and inside of which there is provided an electronic control module, **characterized in that** the replaceable module (6) is mounted in the housing (2) moveably, the drive (8) located in the housing is in motional connection with said replaceable module (6), the replaceable module (6) has at least two segments (6a, 6b , 6c, 6d), each of which is equipped with a moveable diaphragm (10) and thereto assigned puncturing means (11) and blood parameters analyzing means (12), as well as a terminal (13) connecting blood parameters analyzing means (12) with the electronic control module (7), and the electronic control module (7) is equipped with a communication module (7').

2. The device according to claim 1, **characterized in that** the housing (2) has an alarm module (9).

3. The device according to claim 1 or 2, **characterized in that** the replaceable module (6) has the form of a rotatable ring in which there are four segments (6a, 6b, 6c, 6d), wherein the output of each of these segments is protected by a moveable diaphragm (10) and is oriented towards the inside of the ring.

4. The device according to claim 3, **characterized in that** there are four sockets in the ring, forming segments (6a, 6b, 6c, 6d), wherein at the bottom of each od said pockets there is provided with a springy element ended with a puncturing means (11), and wherein an elastic membrane (14) is mounted above the springy element (14) and forming reservoir for blood.

5. The device according to one of the claims from 1 to 4, **characterized in that** the entrance opening (4) of the housing is provided with a moveable closure.

6. The device according to one of the claims 1 to 5, **characterized in that** the electronic control module (7) is provided with a wireless communication module (7'), preferably in the Bluetooth standard.

7. The device according to one of the claims from 1 to 6, **characterized in that** a pneumatic pressure band is mounted in the housing (2), directly in the area of the entrance opening (4).

## Patentansprüche

1. Eine Vorrichtung zur Blutentnahme und Analyse von Blutparametern, umfassend einem Gehäuse mit einer Öffnung, in der sich ein Antrieb befindet, der mit einem auswechselbaren Modul verbunden ist, das mit Stichelementen und Elementen zur Analyse von Blutparametern ausgestattet ist, und in dem ein elektronisches Steuermodul vorgesehen ist, **dadurch gekennzeichnet, dass** das auswechselbare Modul (6) im Gehäuse (2) beweglich gelagert ist und der im Gehäuse eingebaute Antrieb (8) mit dem auswechselbaren Modul (6) beweglich verbunden ist und das auswechselbare Modul (6) mindestens aus zwei Segmenten (6a, 6b, 6c, 6d) aufgebaut ist, von denen jedes mit einer beweglichen Membran (10) und dieser zugeordnetem Stichelement (11) und Blutparameter-Analyseelement (12) sowie einem Anschluss (13) ausgestattet ist, der das Blutparameter-Analyseelement (12) mit dem elektronischen Steuermodul (7) verbindet, und das elektronische Steuermodul (7) mit einem Kommunikationsmodul (7') ausgestattet ist.

2. Eine Vorrichtung nach Anspruch 1, **dadurch gekennzeichnet, dass** das Gehäuse (2) ein Alarmmodul (9) umfasst.

3. Eine Vorrichtung nach Anspruch 1 oder 2, **dadurch gekennzeichnet, dass** das austauschbare Modul (6) in Form eines sich drehenden Rings ausgeführt ist, in dem sich vier Segmente (6a, 6b, 6c, 6d) befinden, wobei der Ausgang jedes dieser Segmente durch eine bewegliche Membran (10) verschlossen und zur Innenseite des Rings hin ausgerichtet ist.

4. Eine Vorrichtung nach Anspruch 3, **dadurch gekennzeichnet, dass** im Ring vier Taschen ausgebildet sind, die Segmente (6a, 6b, 6c, 6d) formen, wobei am Boden jeder dieser Taschen eine Feder eingebaut ist, die mit einem Stichelement (11) abgeschlossen ist, wobei eine elastische Membran (14) über der Feder (14) angebracht ist und so eine Sammeltasche für Blut bildet.

5. Eine Vorrichtung nach einem der Ansprüche 1 bis 4, **dadurch gekennzeichnet, dass** die Eintrittsöffnung (4) des Gehäuses mit einem beweglichen Deckel versehen ist.

6. Eine Vorrichtung nach einem der Ansprüche 1 bis 5, **dadurch gekennzeichnet, dass** das elektronische Steuermodul (7) mit einem drahtlosen Kommunikationsmodul (7') ausgestattet ist, vorzugsweise im Bluetooth-Standard.

7. Eine Vorrichtung nach einem der Ansprüche 1 bis 6, **dadurch gekennzeichnet, dass** ein pneumatisches Druckband im Gehäuse (2), unmittelbar im Bereich der Eintrittsöffnung (4) angebracht ist.

## Revendications

1. Dispositif pour la collecte et l'analyse de paramètres sanguins, comprenant un boîtier avec une ouverture d'entrée, à l'intérieur duquel est prévu un entraînement connecté à un module remplaçable équipé de moyens de ponction et de moyens d'analyse des paramètres sanguins, et à l'intérieur duquel est prévu un module de commande électronique, **caractérisé en ce que** le module remplaçable (6) est monté de manière mobile dans le boîtier (2), l'entraînement (8) situé dans le boîtier est en liaison motrice avec ledit module remplaçable (6), le module remplaçable (6) comporte au moins deux segments (6a, 6b, 6c, 6d), dont chacun est équipé d'un diaphragme mobile (10) auquel sont affectés des moyens de ponction (11) et des moyens d'analyse des paramètres sanguins (12), ainsi qu'un terminal (13) reliant les moyens d'analyse des paramètres sanguins (12) au module de commande électronique (7), et le module de commande électronique (7) est équipé d'un module de communication (7').

2. Dispositif selon la revendication 1, **caractérisé en ce que** le boîtier (2) comporte un module d'alarme (9).

3. Dispositif selon la revendication 1 ou 2, **caractérisé en ce que** le module remplaçable (6) a la forme d'un anneau rotatif dans lequel se trouvent quatre segments (6a, 6b, 6c, 6d), dans lequel la sortie de chacun de ces segments est protégée par un diaphragme mobile (10) et est orientée vers l'intérieur de l'anneau.

4. Dispositif selon la revendication 3, **caractérisé en ce que** l'anneau comprend quatre poches formant des segments (6a, 6b, 6c, 6d), dans lequel au fond de chacune desdites poches est pourvu un élément élastique terminé par un moyen de ponction (11), et dans lequel une membrane élastique (14) est montée au-dessus de l'élément élastique (14) et forme un réservoir pour le sang.

5. Dispositif selon l'une des revendications 1 à 4, **caractérisé en ce que** l'ouverture d'entrée (4) du boîtier est pourvue d'une fermeture mobile.

6. Dispositif selon l'une des revendications 1 à 5, **caractérisé en ce que** le module de commande électronique (7) est pourvu d'un module de communication sans fil (7'), de préférence au standard Bluetooth.

7. Dispositif selon l'une des revendications 1 à 6, **caractérisé en ce qu'**une bande de pression pneumatique est montée dans le boîtier (2) directement dans la zone de l'ouverture d'entrée (4).
